# EUROPEAN PATENT SPECIFICATION

(11) **EP 1 087 980 B1**
(45) Date of publication and mention of the grant of the patent: **29.01.2003**
(21) Application number: 99927894.8
(22) Date of filing: 07.06.1999
(51) Int. Cl.: C07D 501/56, A61K 31/545

(54) **DERIVATIVES OF 3-(2-OXO- 1,3']BIPYRROLIDINYL-3-YLIDENEMETHYL)-CEPHEMS**
3-[2-OXO-(1,3')BIPYRROLIDINYL-3-YLIDEN-METHYL]-CEPHEMDERIVATE
DERIVES DE 3-(2-OXO- 1,3']BIPYRROLIDINYL-3-YLIDENEMETHYL)-CEPHEMES

(30) Priority: 15.06.1998 EP 98110888; 10.09.1998 EP 98117099
(43) Date of publication of application: 04.04.2001
(73) Proprietor: Basilea Pharmaceutica AG, 4102 Binningen (CH)
(72) Inventor: HEBEISEN, Paul, CH-4052 Basle (CH); HUBSCHWERLEN, Christian, F-68200 Durmenach (FR); SPECKLIN, Jean-Luc, F-68680 Kembs-Schaferhof (FR)
(86) International application number: EP9903907
(87) International publication number: WO99065920

(56) References cited:
- EP-A- 0 841 339
- EP-A- 0 849 269

## Description

The present invention is concerned with new compounds of formula I 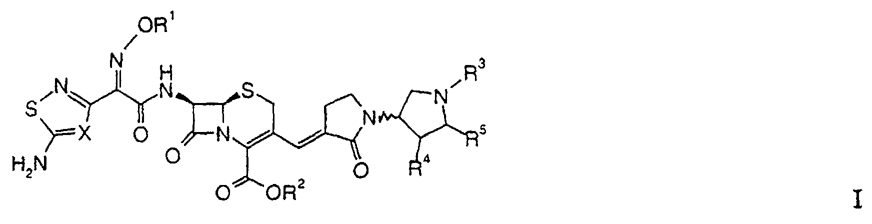 wherein
- R¹: is hydrogen, C₁₋₆-alkyl, optionally substituted by fluoro, or C₃₋₆-cycloalkyl;
- R²: is hydrogen or a group selected from
-CH₂C(=CHR)-COOR, -CH₂OCOR, -CH(R)OCOR,
-CH(R)OCOOR, -CH(OCOR)OCOR, -CH₂COCH₂OCOR and 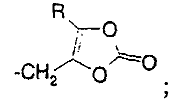
- R³: is hydrogen or group selected from -CH₂C(=CH₂)-COOR, -COOCH₂C(=CHR)-COOR, -COOCH₂OCOR, -COOCH(R)OCOR, -COOCH(R)OCOOR, -COOCH(OCOR)OCOR, -COOCH₂COCH₂OCOR, and 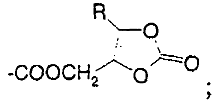 with the proviso that one of R² and R³ is hydrogen and the other of R² and R³ is different from hydrogen,
- R: is hydrogen or C₁₋₆-alkyl;
- R⁴: is hydrogen or hydroxy,
- R⁵: is hydrogen or ω-hydroxyalkyl; and
- X: is CH or N,
as well as pharmaceutically acceptable salts of said compounds and hydrates of the compounds of formula I and of their salts.

Vinylpyrrolidinon cephaslosporin derivatives are known to be useful antibiotic compounds as described in EP A-0 841 339, furthermore compounds of formula A 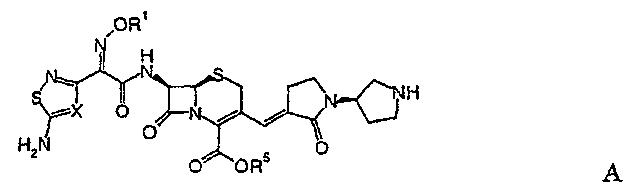 wherein
R¹ and X are as defined above and R⁵ is hydrogen, and pharmaceutically acceptable salts thereof,
especially (6R,7R)-7-[(Z)-2-(5-amino-[1,2,4]thiadiazol-3-yl)-2-hydroxyimino-acetylamino]-8-oxo-3-[(E)-(R)-2-oxo- [1,3']bipyrrolidinyl-3-ylidenemethyl]-5-thia-1-aza-bicyclo[4.2.0]oct-2-ene-2-carboxylic acid are potent antibacterial agents with activity against methicillin resistant staphylococci, both *in vitro* and *in vivo.* However, these compounds have limited solubility, not allowing bolus injections. It is therefore necessary to find derivatives of the compounds A to render these compounds suitable for parenteral and intramuscular application.

From J. Med. Chem. (1996), 39(2), 480-6; US patent no. 5 466 811; Bioorganic and Medicinal Chem. Lett. 1997,7,2909-2912; US patent no. 5 610 314 (oxodioxolenyl)methyl carbamates are known to form derivatives of amines e.g. for fibrinogen receptor antagonists, ampicillin, norfloxacin and other pharmaceuticals. Furthermore, 2-(alkyloxycarbonyl)-2-alkylideneethyl esters have been described as prodrugs of carboxylic acids for cephalosporins, J. Antibiot. (1992), 45(8), 1358-64. Both types of derivatives have been used to improve the oral bioavailability of the corresponding drugs.

It has now been found that the compounds of formula I exhibit better solubility in water and buffers at physiological pH. *In vitro* and *in vivo* they were readily converted to compounds of formula **A** and can therefore be used for parenteral and intramuscular application forms. The invention is thus also concerned with pharmaceutical preparations containing a compound of formula I and a therapeutically inert carrier.

As used herein "pharmaceutically acceptable salts" useful in this invention include salts derived from metals, salts from amino acids and salts of mineral or organic acids. Examples of preferred metal salts are those derived from the alkali metals, for example, lithium (Li⁺), sodium (Na⁺) and potassium (K⁺). Especially preferred is sodium.-Other salts are derived from amino acids such as, for example, salts with arginine or lysine. Examples of salts of mineral acids are for example hydrochlorides, sulphates or phosphates, and examples of salts of organic acids are mesylates (methylsulfonic acid salts), napsylates (naphthtene-2-sulfonic acid salts), besylates (benzenesulfonic acid salts), maleates, salicylates, tartrates, lactates, citrates, benzoates, succinates, acetates and the like. Especially preferred are chlorides, sulfates, phosphates, lactates and mesylates.

In the formulas represented herein, when substituents are illustrated as joined to the nucleus a solid line ( 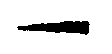 ), indicates that the substituent is in the β-orientation, that is, above the plane of the molecule, a broken line ( 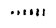 ), indicates that the substituent is in the α-orientation, that is, below the plane of the molecule whereas the ...... line ( 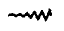 ) indicates that the bond is either in α- or in β-orientation.

The term "C₁₋₆-alkyl, optionally substituted by fluoro" refers to both straight and branched chain saturated hydrocarbon groups having 1 to 6 and preferably 1 to 4 carbon atoms, for example, methyl, ethyl, n-propyl, isopropyl, tertiary butyl and the like, these group may be substituted by one or more fluorine atoms as e.g. in fluoromethyl or trifluoromethyl.

As used herein, the term "ω-hydroxyalkyl" refers to both straight and branched chain saturated hydrocarbon groups as defined above bearing a hydroxy group in the terminal position, e.g. hydroxymethyl, 2-hydroxyethyl, 3-hydroxypropyl, preferably hydroxymethyl.

By the term "C₃₋₆-cycloalkyl" is meant a 3-6 membered saturated carbocyclic moiety, e.g., cyclopropyl, cyclobutyl, cyclopentyl and cyclohexyl, in particular cyclopentyl.

Especially preferred compounds of formula I are the compounds of formula 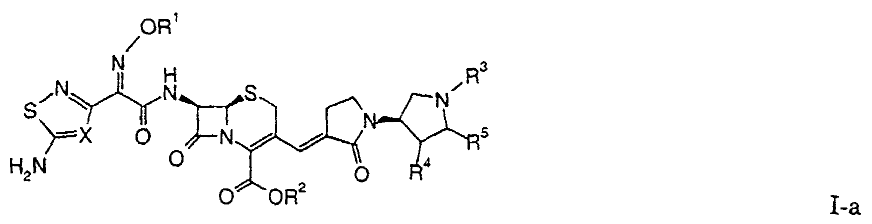 wherein, R¹ R² R³ and X are as defined above and R⁴ and R⁵ are hydrogen, as well as pharmaceutically acceptable salts of said compounds and hydrates of the compounds of formula I-a and of their salts.

Further preferred compounds are epimers and diastereoisomers of formula I-a.

Preferred compounds of formula I and formula I-a are compounds wherein R¹ is hydrogen, X is N and R² is hydrogen and R³ is a group chosen from
-CH₂C(=CH₂)-COOCH₂CH₃,
-COO-CH₂C(=CHCH₂CH₃)-COOCH₂CH(CH₃)₂,
and particularly

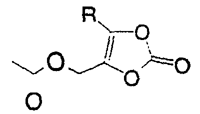

Further preferred compounds of formula I are compounds wherein R1 and R3 are hydrogen, X is N and R2 is a group chosen from
-CH2C(=CHCH2CH3)-COOCH2CH(CH3)2, -CH2C(=CH2)-COOCH2CH3, -CH2OCOC(CH3)3, -CH(CH3)OCOCH3, -CH(CH3)OCOOCH2CH3, -CH(OCOCH3)OCOCH3, -CH2COCH2OCOCH3, and

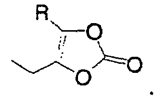

Especially preferred compounds of formula I and I-a are
(6R,7R)-7-[(Z)-2-(5-amino-[1,2,4]thiadiazol-3-yl)-2-hydroxyimino-acetylamino]-3-[(E)-(R)-1'-(5-methyl-2-oxo-[1,3]dioxol-4-ylmethoxycarbonyl)-2-oxo-[1,3']bipyrrolidinyl-3-ylidenemethyl]-8-oxo-5-thia-1-aza-bicyclo[4.2.0]oct-2-ene-2-carboxylic acid
(6R,7R)-7-[(Z)-2-(5-amino-[1,2,4]thiadiazol-3-yl)-2-hydroxyimino-acetylamino]-3-[(E)-(R)-1'-(5-ethyl-2-oxo-[1,3]dioxol-4-ylmethoxycarbonyl)-2-oxo-[1,3']bipyrrolidinyl-3-ylidenemethyl)-8-oxo-5-thia-1-aza-bicyclo[4.2.0]oct-2-ene-2-carboxylic acid sodium salt (1:1)
(6R,7R)-7-[(Z)-2-(5-Amino-[1,2,4]thiadiazol-3-yl)-2-hydroxyimino-acetylamino]-3-[(E)-(R)-1'-(2-oxo-5-propyl-[1,3]dioxol-4-ylmethoxycarbonyl)-2-oxo-[1,3']bipyrrolidinyl-3-ylidenemethyl]-8-oxo-5-thia-1-aza-bicyclo[4.2.0]oct-2-ene-2-carboxylic acid sodium salt (1:1)
(6R,7R)-7-[(Z)-2-(5-amio-[1,2,4]thiadiazol-3-yl)-2-hydroxyimino-acetylamino]-3-[(E)-(R)-1'-(5-isopropyl-2-oxo-[1,3]dioxol-4-ylmethoxycarbonyl)-2-oxo-[1,3']bipyrrolidinyl-3-ylidenemethyl]-8-oxo-5-thia-1-aza-bicyclo[4.2.0]oct-2-ene-2-carboxylic acid sodium salt (1:1)
(6R,7R)-7-[(Z)-2-(5-amino-[1,2,4]thiadiazol-3-yl)-2-hydroxyimino-acetylamino]-3-[(E)-(R)-1'-( 5-tert-butyl-2-oxo-[1,3]dioxol-4-ylmethoxycarbonyl)-2-oxo-[1,3']bipyrrolidinyl-3-ylidenemethyl]-8-oxo-5-thia-1-aza-bicyclo[4.2.0]oct-2-ene-2-carboxylic acid sodium salt (1:1)
(6R,7R)-7-[(Z)-2-(5-Amino-[1,2,4]thiadiazol-3-yl)-2-hydroxyimino-acetylamino]-3-[(E)-(R)-1'-(E-2-isobutoxycarbonyl-pent-2-enyloxycarbonyl)-2-oxo-[1,3']bipyrrolidinyl-3-ylidenemethyl]-8-oxo-5-thia-1-aza-bicyclo[4.2.0]oct-2-ene-2-carboxylic acid sodium salt (1:1)
(6R,7R)-7-[(Z)-2-(5-Amino-[1,2,4]thiadiazol-3-yl)-2-hydroxyimino-acetylamino]-3-[(E)-(R)-1'-(2-ethoxycarbonyl-allyl)-2-oxo-[1,3']bipyrrolidinyl-3-ylidenemethyl]-8-oxo-5-thia-1-aza-bicyclo[4.2.0]oct-2-ene-2-carboxylic acid sodium salt (1:1)
(6R,7R)-7-[(Z)-2-(5-Amino-[1,2,4]thiadiazol-3-yl)-2-hydroxyiminoacetylaminol-3-[(E)-5'-hydroxymethyl-1'-(5-methyl-2-oxo-[1,3]dioxol-4-ylmethoxycarbonyl)-2-oxo-[1,3']bipyrrolidinyl-3-ylidenemethyl]-8-oxo-5-thia-1-aza-bicyclo[4.2.0]oct-2-ene-2-carboxylic acid sodium salt
(6R,7R)-7-[(Z)-2-(5-amino-[1,2,4]thiadiazol-3-yl)-2-hydroxyimino-acetylamino]-3-[(E)-(3'S,4'S)- and -(3'R,4'R)-4'-hydroxy-1'-(5-methyl-2-oxo-[1,3]dioxol-4-ylmethoxycarbonyl)-2-oxo-[1,3']bipyrrolidinyl-3-ylidenemethyl]-8-oxo-5-thia-1-aza-bicyclo[4.2.0]oct-2-ene-2-carboxylic acid sodium salt

The compounds of formula I and I-a as well as their salts can be hydrated. The hydration can be effected in the course of the manufacturing process or can occur gradually as a result of hygroscopic properties of an initially anhydrous product.

The compounds of the present invention are useful as antibiotics having potent and broad antibacterial activity; especially against methicillin resistant Staphylococci (MRSA) and Pseudomonas *aeruginosa.*

The products in accordance with the invention can be used as medicaments, for example, in the form of pharmaceutical preparations for parenteral administration, and for this purpose are preferably made into preparations as lyophilisates or dry powders for dilution with customary agents, such as water or isotonic common salt or carbohydrate (e.g. glucose) solution.

Depending on the nature of the pharmacologically active compound the pharmaceutical preparations can contain the compound for the prevention and treatment of infectious diseases in mammals, humans and non-humans. A daily dosage of about 10 mg to about 4000 mg, especially about 50 mg to about 3000 mg, is usual, with those of ordinary skill in the art appreciating that the dosage will depend also upon the age, conditions of the mammals, and the kind of diseases being prevented or treated. The daily dosage can be administered in a single dose or can be divided over several doses. An average single dose of about 50 mg, 100 mg, 250 mg, 500 mg, 1000 mg, and 2000 mg can be contemplated.

The compounds of the formula I and I-a in accordance with the invention as well as their pharmaceutically acceptable salts, hydrates, or readily hydrolyzable esters can for example be prepared in accordance to procedures given below:

Compounds of formula I and I-a wherein R² is hydrogen and R³ is a group -COOCH₂C(=CHR)-COOR, -COOCH₂OCOR, -COOCH(R)OCOR, -COOCH(R)OCOOR, -COOCH(OCOR)OCOR, -COOCH₂COCH₂OCOR or 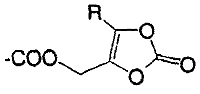 can be prepared according to Scheme 1 by acylation of compounds of formula A with the corresponding carbonic acid 4-nitrophenyl ester. 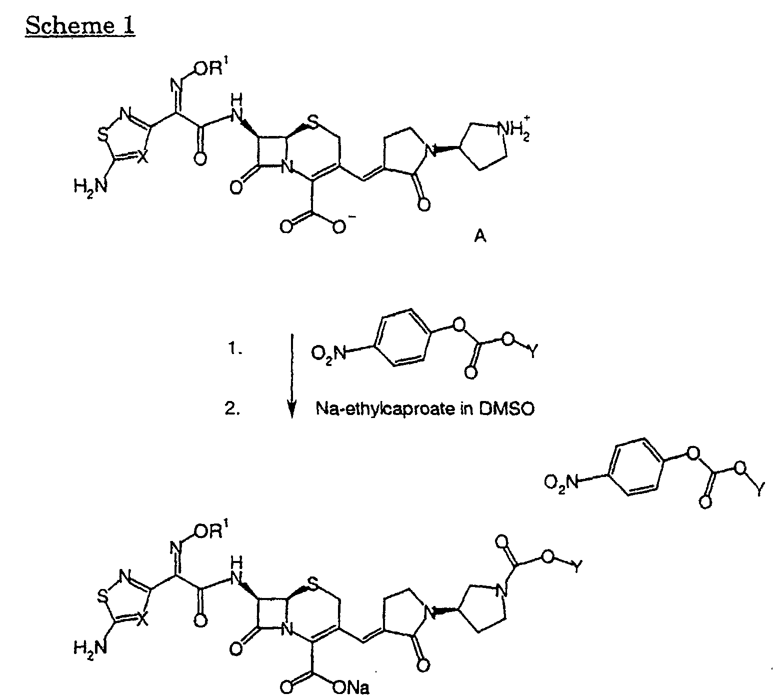 wherein R¹ and R are as defined above and Y is a group -CH₂C(=CHR)-COOR, -CH₂OCOR, -CH(R)OCOR, -CH(R)OCOOR, -CH(OCOR)OCOR, -CH₂CO CH₂OCOR, or

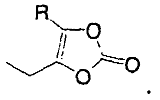

For the preparation of compounds of formula I and I-a wherein R³ is -CH₂C(=CH₂)-COOR a compound of formula A is reacted with 2-(4-nitrophenoxycarbonyloxymethyl)-acrylic acid ethyl ester. This reaction proceeds with loss of carbon dioxide, cf. scheme 2:

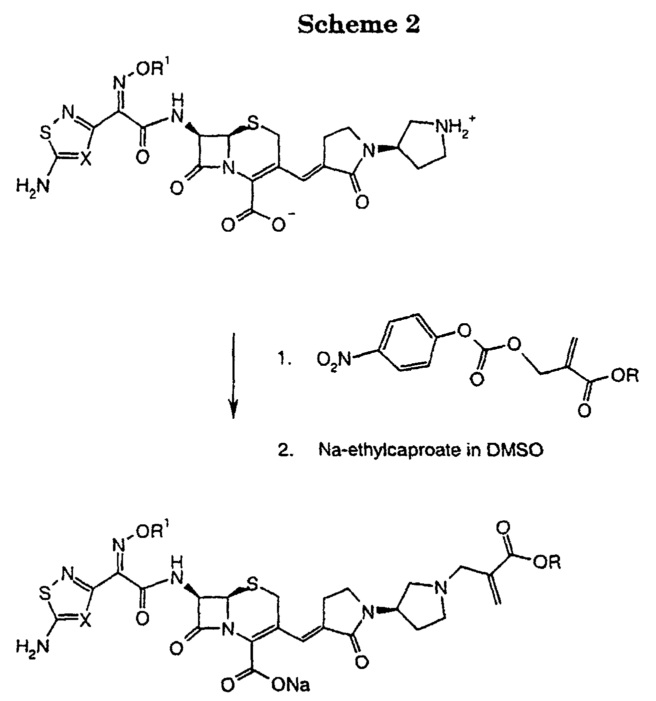

The synthesis of the compounds of formula I and I-a, wherein R³ is hydrogen, (Scheme 3) is accomplished via alkylation of the carboxylate, preferably at the stage of the fully protected compound of formula A. Protecting groups for R¹ are preferably, the trityl and in position R³ tert.-butyloxycarbonyl (BOC). Removal of the tert.-butyloxycarbonyl and trityl-protecting groups is accomplished by conventional methods and the desired compounds of formula I and I-a are isolated as the hydrochloride salts by precipitation from dioxane. 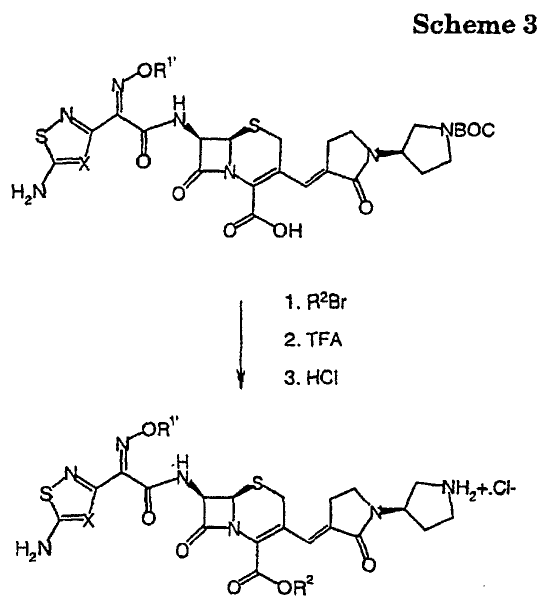 wherein R^{1'} is C₁₋₆-alkyl, optionally substituted by fluoro, or C₃₋₆-cycloalkyl or a protecting group, preferably trityl, and BOC is tert.-butyloxycarbonyl

Compounds of formula A are known compounds and can be prepared according to the methods described in EP A-0 849 269.

The alkylating agents, i.e. the compounds R²Br or R²Cl are known compounds, some are commercially available.

The following examples further illustrate the invention, however, without limiting its scope.

### Examples

**1.1. Synthesis of (6R,7R)-7-[(Z)-2-(5-amino-[1,2,4]thiadiazol-3-yl)-2-hydroxyimino-acetylamino]-3-[(E)-(R)-1'-(5-methyl-2-oxo-[1,3]dioxol-4-ylmethoxycarbonyl)-2-oxo-[1,3']bipyrrolidinyl-3-ylidenemethyl]-8-oxo-5-thia-1-aza-bicyclo[4.2.0]oct-2-ene-2-carboxylic acid** 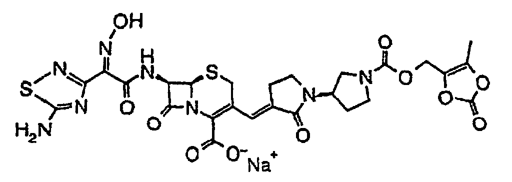
   To a solution of 13.2 g (44.72 mmol) of carbonic acid 5-methyl-2-oxo-[1,3]dioxol-4-ylmethyl ester 4-nitro-phenyl ester in 200 ml of dimethylsulfoxide are added 20.0 g of (6R,7R)-7-[(Z)-2-(5-amino-[1,2,4]thiadiazol-3-yl)-2-hydroxyimino-acetylamino]-8-oxo-3-[(E)-(R)-2-oxo-[1,3']bipyrrolidinyl-3-ylidenemethyl]-5-thia-1-aza-bicyclo[4.2.0]oct-2-ene-2-carboxylic acid. The mixture is stirred under argon at room temperature for 4 hours and 1000 ml of acetone are added. The slightly turbid solution is clarified by filtration over a fluted filter. To the clear motherliquour are added 34.0 ml (34 mmol) of a 1N solution of sodium-2-ethylcaproate in acetone at room temperature during 20 minutes. The slightly yellow suspension is stirred for 10 minuntes at room temperature, the solid is collected by filtration, washed with 1000 ml of acetone and 1000 ml of n-pentane and dried under high vacuum. The product is suspended in 600 ml of acetone and stirred at room temperature for 2 hours. The product is collected by filtration and dried under high vacuum to yield 23.94 g of the title compound as an off-white powder.
   MS(ISP): M+H⁺=691.3;M+NH4⁺=708.2;M+Na⁺=713.1
   In a similar manner, the following carbamates are prepared from the corresponding mixed carbonates:
**1.2. (6R,7R)-7-[(Z)-2-(5-Amino-[1,2,4]thiadiazol-3-yl)-2-hydroxyimino-acetylamino]-3-[(E)-(R)-1'-(5-ethyl-2-oxo-[1,3]dioxol-4-ylmethoxycarbonyl)-2-oxo-[1,3']bipyrrolidinyl-3-ylidenemethyl]-8-oxo-5-thia-1-aza-bicyclo[4.2.0]oct-2-ene-2-carboxylic acid sodium salt (1:1)** 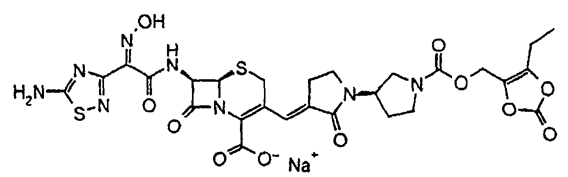 MS (ISP): M+H⁺=705.2,M+NH₄⁺=722.3,M+Na⁺=727.2
**1.3. (6R,7R)-7-[(Z)-2-(5-Amino-[1,2,4]thiadiazol-3-yl)-2-hydroxyimino-acetylamino]-3-[(E)-(R)-1'-(2-oxo-5-propyl-[1,3]dioxol-4-ylmethoxycarbonyl)-2-oxo-[1,3']bipyrrolidinyl-3-ylidenemethyl]-8-oxo-5-thia-1-aza-bicyclo[4-2.0]oct-2-ene-2-carboxylic acid sodium salt (1:1)** 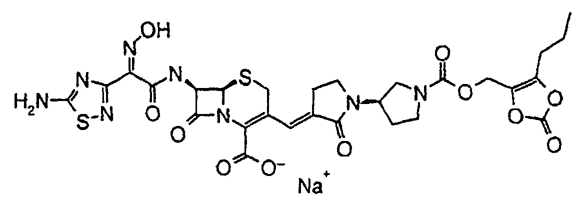 MS (ISP): M+H⁺=719.3,M+NH₄⁺=736.2,M+Na⁺=741.2
**1.4. (6R,7R)-7-[(Z)-2-(5-Amino-[1,2,4]thiadiazol-3-yl)-2-hydroxyimino-acetylamino]-3-[(E)-(R)-1'-(5-isopropyl-2-oxo-[1,3]dioxol-4-ylmethoxycarbonyl)-2-oxo-[1,3']bipyrrolidinyl-3-ylidenemethyl]-8-oxo-5-thia-1-aza-bicyclo[4.2.0]oct-2-ene-2-carboxylic acid sodium salt (1:1)** 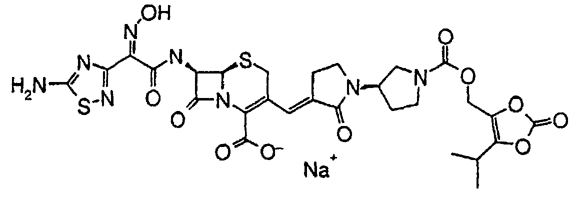 MS (ISP): M+H⁺=719.3,M+NH₄⁺=736.2,M+Na⁺=741.2
**1.5. (6R,7R)-7-[(Z)-2-(5-Amino-[1,2,4]thiadiazol-3-yl)-2-hydroxyimino-acetylamino]-3-[(E)-(R)-1'-(5-tert-butyl-2-oxo-[1,3]dioxol-4-ylmethoxycarbonyl)-2-oxo-[1,3']bipyrrolidinyl-3-ylidenemethyl]-8-oxo-5-thia-1-aza-bicyclo[4.2.0]oct-2-ene-2-carboxylic acid sodium salt (1:1)** 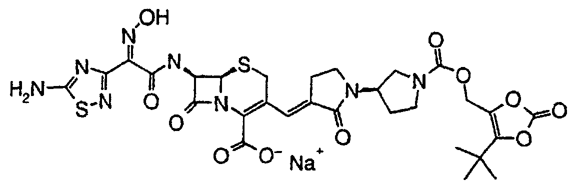 MS (ISP): M+H⁺=733.2,M+NH₄⁺=750.4
**1.6. (6R,7R)-7-[(Z)-2-(5-Amino-[1,2,4]thiadiazol-3-yl)-2-hydroxyimino-acetylamino]-3-[(E)-(R)-1'-(E-2-isobutoxycarbonyl-pent-2-enyloxycarbonyl)-2-oxo-[1,3']bipyrrolidinyl-3-ylidenemethyl]-8-oxo-5-thia-1-aza-bicyclo[4.2.0]oct-2-ene-2-carboxylic acid sodium salt (1:1)** 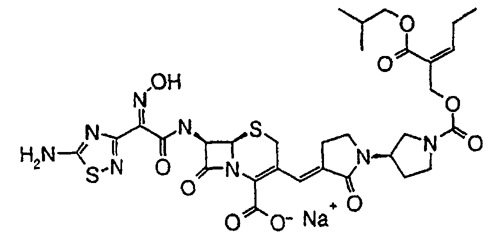 MS (ISP): M+H⁺=747.4,M+NH₄⁺=764.3,M+Na⁺=769.3
**1.7. (6R,7R)-7-[(Z)-2-(5-Amino-[1,2,4]thiadiazol-3-yl)-2-hydroxyimino-acetylamino]-3-[(E)-(R)-1'-(2-ethoxycarbonyl-allyl)-2-oxo-** **[1,3']bipyrrolidinyl-3-ylidenemethyl]-8-oxo-5-thia-1-azabicyclo[4.2.0]oct-2-ene-2-carboxylic acid sodium salt (1:1)** 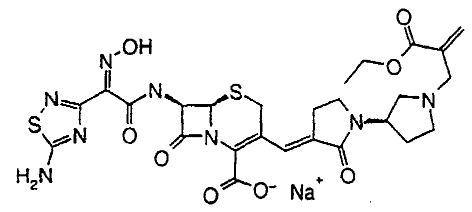 MS (ISP): M+H⁺=647.4
**1.8. (6R,7R)-7-[(Z)-2-(5-Amino-[1,2,4]thiadiazol-3-yl)-2-hydroxyimino-acetylamino]-3-[(E)-(3'R,5'S)-5'-hydroxymethyl-1'-(5-methyl-2-oxo-[1,3]dioxol-4-ylmethoxycarbonyl)-2-oxo-[1,3']bipyrrolidinyl-3-ylidenemethyl]-8-oxo-5-thia-1-aza-bicyclo[4.2.0]oct-2-ene-2-carboxylic acid sodium salt** 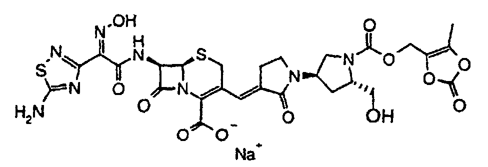 MS(ISP): M+H⁺= 721.3;M+NH4⁺=738.2;M+Na⁺=743.2
**1.9 1:1 Mixture of (6R,7R)-7-[(Z)-2-(5-amino-[1,2,4]thiadiazol-3-yl)-2-hydroxyimino-acetylamino]-3-[(E)-(3'S,4'S)- and -(3'R,4'R)-4'-hydroxy-1'-(5-methyl-2-oxo-[1,3]dioxol-4-ylmethoxycarbonyl)-2-oxo-[1,3']bipyrrolidinyl-3-ylidenemethyl]-8-oxo-5-thia-1-aza-bicyclo[4.2.0]oct-2-ene-2-carboxylic acid sodium salt (1:1)** 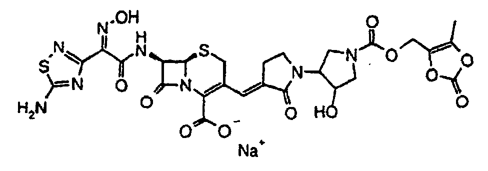 MS(ISN):M-H⁺=705.3

Preparation of the starting materials for examples 1.7. and 1.6.:
**a) 2-(4-Nitro-phenoxycarbonyloxymethyl)-acrylic acid ethyl ester** 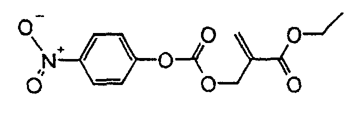
   A mixture of 2.60 g of 2-hydroxymethyl-acrylic acid ethyl ester (0.020 mol) and 4.0 g of 4-nitrophenyl-chloroformate (0.020 mol) in 70 ml dichloromethane is treated with a solution of 5.5 ml (0.030 mol) of ethyl-diisopropylamine in 55 ml of dichloromethane at 0°C for two hours. The mixture is hydrolyzed with 10% potassium bicarbonate, the phases are separated and the product contained in the organic phase is purified by chromatography on silica gel using a 4:1 mixture of n-hexane and ethyl acetate as eluent. The product fractions are collected and evaporated yielding 3.5g (60%) of the title compound as a white crystalline solid with melting point 42-43°C after crystallisation from t-butylmethyl ether and n-hexane.
**b) 2-(4-Nitro-phenoxycarbonyloxymethyl)-pent-2-enoic acid isobutyl ester** 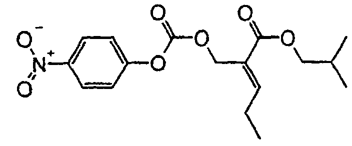 is prepared according to the procedure described above. A yellowish oil is obtained.
   **MS (EI): M+H**^{**+**}**=352 M-C**_{**4**}**H**_{**7**}**O = 278**

### Example 2

**2.1.**
   **a) Synthesis of (6R,7R)-7-[(Z)-2-(5-amino-[1,2,4]thiadiazol-3-yl)-2-trityloxyimino-acetylamino]-3-[(E)-(R)-1'-tert-butoxycarbonyl-2-oxo-[1,3']bipyrrolidinyl-3-ylidenemethyl]8-oxo-5-thia-1-aza-bicyclo[4.2.0]oct-2-ene-2-carboxylic acid** 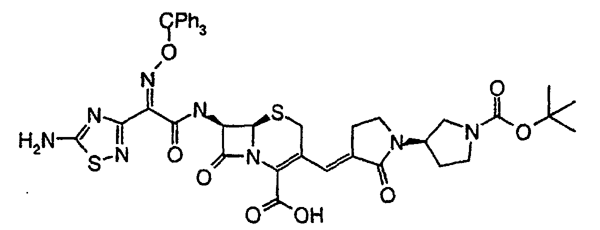
      To a solution of 1.67g (1.965 mmol) of (6R,7R)-7-[(Z)-2-(5-amino-[1,2,4]thiadiazol-3-yl)-2-trityloxyimno-acetylammino]-8-oxo-3-[(E)-(R)-2-oxo-[1,3']bipyrrolidinyl-3-ylidenemethyl)-5-thia-1-aza-bicyclo[4.2.0]oct-2-ene-2-carboxylic acid hydrochloride salt (1:2) in 20.0 ml of dimethylformamide are added 0.56 ml (4.00 mmol) of triethylamine and 0.554 g di-tert-butyldicarbonate and the mixture is stirred at room temperature for 1.5 hours. The mixture is diluted with 500 ml of ethyl acetate and 100 ml water. The pH is adjusted to 2 by the addition of 1N hydrochloric acid. The phases are separated and the organic phase is washed twice with 100 ml of water, clarified by filtration over a fluted filter and concentrated. 100 ml of diethyl ether are added and the resulting suspension is stirred at room temperature for 1 hour. The solid is collected by filtration, washed with diethyl ether and dried, yielding 1.31g (76% ) of the title compound as a beige powder.
      MS (ISP): (M+H)⁺=877.4 (M+NH₄)⁺=894.4 (M+Na)⁺=899.4
   **b) Synthesis of (6R,7R)-7-[(Z)-2-(5-amino-[1,2,4]thiadiazol-3-yl)-2-hydroxyimino-acetylamino]-8-oxo-3-[(E)-(R)-2-oxo-[1,3']bipyrrolidinyl-3-ylidenemethyl]-5-thia-1-aza-bicyclo[4.2.0]oct-2-ene-2-carboxylic acid (E)-2-isobutoxycarbonyl-pent-2-enyl ester hydrochloride (1:1.6)** 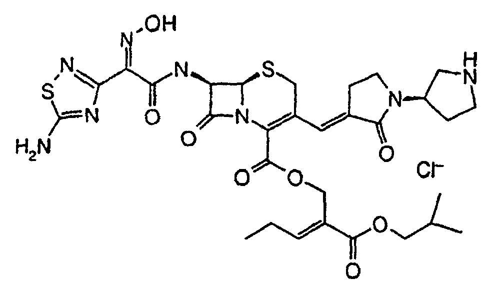
      To a solution of 0.150 g (0.171 mmol) of (6R,7R)-7-[(Z)-2-(5-amino-[1,2,4]thiadiazol-3-yl)-2-trityloxyimino-acetylamino]-3-[(E)-(R)-1'-tert-butoxycarbonyl-2-oxo-[1,3']bipyrrolidinyl-3-ylidenemethyl]8-oxo-5-thia-1-aza-bicyclo[4.2.0]oct-2-ene-2-carboxylic acid in 1 ml of dimethylsulfoxide are added 18.7 mg of (0.163 mmol) 1,1-3,3-tetramethyl guanidine and 64 mg (0.257 mmol) of isobutyl (Z)-2-(bromomethyl)-2-pentenoate, and the mixture is stirred at room temperature for 20 minutes. The solvent is evaporated under high vacuum and the residue purified by chromatography on MCI-gel using a gradient of 40 to 100% of acetonitrile in water as eluent. The product fractions are collected and the organic solvent is removed by evaporation. The product is extracted from the remaining aqueous phase with dichloromethane. The combined organic phases are dried over magnesium sulfate, evaporated to dryness and triturated with tert-butylmethyl ether to yield 130 mg (0.125 mmol) beige crystals with melting point 158-159°C which are dissolved in 1.2 ml of dichloromethane. To this solution are added 0.0366 ml (0.23 mmol) of triethylsilane and 0.38 ml of trifluoroacetic acid at 0°C and the mixture is stirred for 1 hour. To the resulting clear solution 2.0 ml of dioxane are added and the mixture is concentrated under vacuum. The residue is dissolved in 2.0 ml of dioxane and 0.182 ml of a 1.9 N solution of hydrochloric acid in dioxane are added with stirring at room temperature. The precipitate is collected by filtration washed with dioxane and acetone and dried yielding 85 mg of the title compound as beige crystals melting at 139-140°C (dec). HPLC: 99.%(area) MS: M+H⁺=703.3
      In a similar manner the following prodrug-ester compounds are prepared starting from the corresponding alkyl halides:
**2.2. (6R,7R)-7-[(Z)-2-(5-amino-[1,2,4]thiadiazol-3-yl)-2-hydroxyimino-acetylamino]-8-oxo-3-[(E)-(R)-2-oxo-[1,3']bipyrrolidinyl-3-ylidenemethyl]-5-thia-1-aza-bicyclo[4.2.0]oct-2-ene-2-carboxylic acid 2,2-dimethyl-propionyloxymethyl ester hydrochloride** 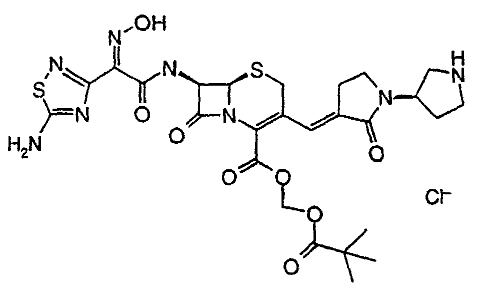 MS (ISP): M+H⁺=649.3
**2.3. (6R,7R)-7-[(Z)-2-(5-amino-[1,2,4]thiadiazol-3-yl)-2-hydroxyimino-acetylamino]-8-oxo-3-[(E).(R)-2-oxo-[1,3']bipyrrolidinyl-3-ylidenemethyl]-5-thia-1-aza-bicyclo[4.2.0]oct-2-ene-2-carboxylic acid 5-methyl-2-oxo-[1,3]dioxol-4-ylmethyl ester hydrochloride (1:1.5)** 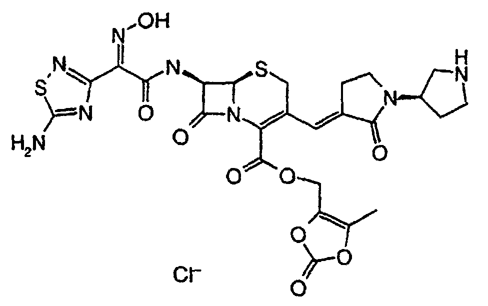 MS (ISP): M+H⁺=647.3
**2.4. 1:1 Mixture of (6R,7R)-7-[(Z)-2-(5-amino-[1,2,4]thiadiazol-3-yl)-2-hydroxyimino-acetylamino]-8-oxo-3-[(E)-(R)-2-oxo-[1,3']bipyrrolidinyl-3-ylidenemethyl]-5-thia-1-aza-bicyclo[4.2.0]oct-2-ene.2-carboxylic acid (R)- and (S)-1-acetoxy-ethyl ester hydrochloride (1:1.2)** 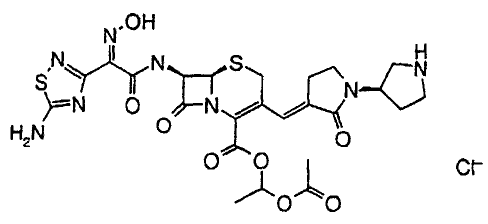 MS (ISP): M+H⁺=621.3
**2.5. 1:1 Mixture of (6R,7R)-7-[(Z)-2-(5-amino-[1,2,4]thiadiazol-3-yl)-2-hydroxyimino-acetylamino]-8-oxo-3-[(E)-(R)-2-oxo-[1,3']bipyrrolidinyl-3-ylidenemethyl]-5-thia-1-aza-bicyclo[4.2.0]oct-2-ene-2-carboxylic acid (R)- and (S)-1-ethoxycarbonyloxy-ethyl ester hydrochloride (1:1.4)** 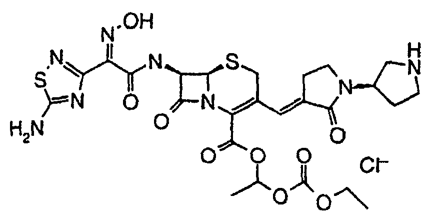 MS (ISP): M+H⁺=651.2
**2.6. 1:1 Mixture of (6R,7R)-7-[(Z)-2-(5-amino-[1,2,4]thiadiazol-3-yl)-2-hydroxyimino-acetylamino]-8-oxo-3-[(E)-(R)-2-oxo-[1,3']bipyrrolidinyl-3-ylidenemethyl]-5-thia-1-aza-bicyclo[4.2.0]oct-2-ene-2-carboxylic acid (R)- and (S)-1-acetoxy-2-oxo-propyl ester hydrochloride (1:1.4)** 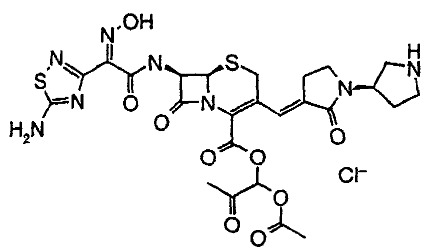 MS (ISP): M+H⁺=649
**2.7. (6R,7R)-7-[(Z)-2-(5-Amino-[1,2,4]thiadiazol-3-yl)-2-hydroxyimino-acetylamino]-8-oxo-3-[(E)-(R)-2-oxo-[1,3']bipyrrolidinyl-3-ylidene** **methyl]-5-thia-1-aza-bicyclo[4.2.0]oct-2-ene-2-carboxylic acid 3-acetoxy-2-oxo-propyl ester hydrochloride (1:1.65)** 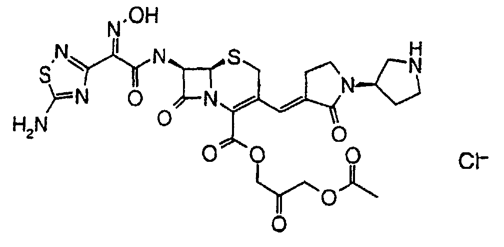 MS (ISP): M+H⁺=649.2

## Claims

1. Compounds of formula I 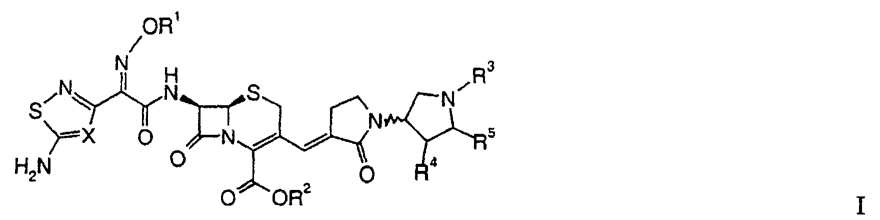 wherein
R¹ is hydrogen, C₁₋₆-alkyl, optionally substituted by fluoro, or C₃₋₆-cycloalkyl;
R² is hydrogen or a group selected from
-CH₂C(=CHR)-COOR, -CH₂OCOR, -CH(R)OCOR, -CH(R)OCOOR, -CH(OCOR)OCOR, -CH₂COCH₂OCOR and 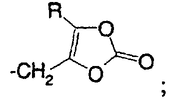
R³ is hydrogen or group selected from -CH₂C(=CH₂)-COOR, -COOCH₂C(=CHR)-COOR, -COOCH₂OCOR, -COOCH(R)OCOR, -COOCH(R)OCOOR, -COOCH(OCOR)OCOR, -COOCH₂COCH₂OCOR, and 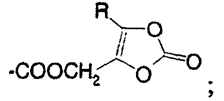 with the proviso that one of R² and R³ is hydrogen and the other of R² and R³ is different from hydrogen,
R is hydrogen or C₁₋₆-alkyl;
R⁴ is hydrogen or hydroxy,
R⁵ is hydrogen or ω-hydroxyalkyl; and
X is CH or N,
as well as pharmaceutically acceptable salts of said compounds and hydrates of the compounds of formula I and of their salts.

2. Compounds according to claim 1 of formula I-a 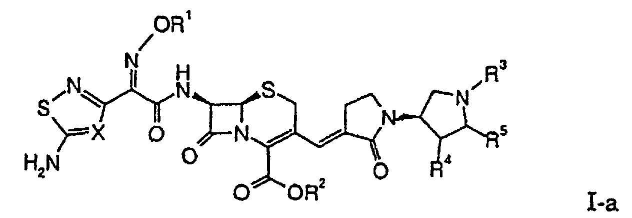 wherein R¹, R², R³ and X are as defined in claim 1 and R⁴ and R⁵ are hydrogen,
as well as pharmaceutically acceptable salts of said compounds and hydrates of the compounds of formula I-a and of their salts.

3. Compounds according to claim 1 or 2, wherein R¹ is hydrogen, X is N, R² is hydrogen and R³ is a group
-COO-CH₂C(=CHCH₂CH₃)-COOCH₂CH(CH₃)₂,
-CH₂C(=CH₂)-COOCH₂CH₃,
or

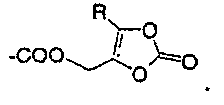

4. Compounds according to claim 1 or 2 wherein R¹ and R³ are hydrogen, X is N and R² is -CH₂C(=CHCH₂CH₃)-COOCH₂CH(CH₃)₂, -CH₂C(=CH₂)-COOCH₂CH₃, -CH₂OCOC(CH₃)₃, -CH(CH₃)OCOCH₃, -CH(CH₃)OCOOCH₂CH₃, -CH(OCOCH₃)OCOCH₃, -CH₂COCH₂OCOCH₃, or

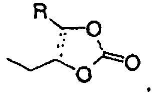

5. The compound according to claims 1 and 2
(6R,7R)-7-[(Z)-2-(5-amino-[1,2,4]thiadiazol-3-yl)-2-hydroxyimino-acetylamino]-3-[(E)-(R)-1'-(5-methyl-2-oxo-[1.3]dioxol-4-ylmethoxycarbonyl)-2-oxo-[1,3']bipyrrolidinyl-3-ylidenemethyl]-8-oxo-5-thia-1-aza-bicyclo[4.2.0]oct-2-ene-2-carboxylic acid

6. The compound according to claims 1 and 2
(6R,7R)-7-[(Z)-2-(5-amino-[1,2,4]thiadiazol-3-yl)-2-hydroxyimino-acetylamino]-3-[(E)-(R)-1'-(5-ethyl-2-oxo-[1,3]dioxol-4-ylmethoxycarbonyl)-2-oxo-[1,3']bipyrrolidinyl-3-ylidenemethyl]-8-oxo-5-thia-1-aza-bicyclo[4.2.0]oct-2-ene-2-carboxylic acid sodium salt (1:1)

7. Compounds as in any one of claims 1 to 6 for use as pharmaceutically active substances, particularly for the treatment and prophylaxis of infectious diseases.

8. A pharmaceutical preparation containing a compound according to any one of claims 1 to 6 and a therapeutically inert carrier, particularly for the treatment and prophylaxis of infectious diseases.

9. The use of the compounds according to any one of claims 1 to 6 for the manufacture of medicaments for the treatment and prophylaxis of infectious diseases.

## Patentansprüche

1. Verbindungen der Formel I 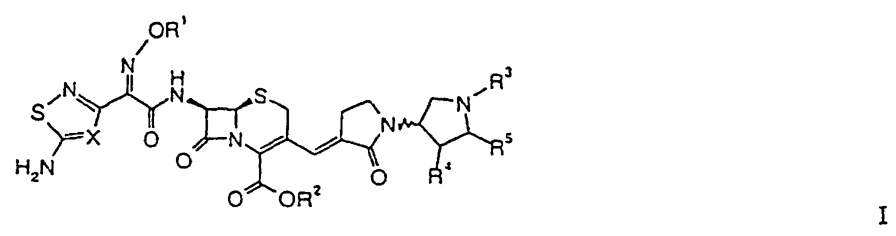 worin
R¹ Wasserstoff, ein gegebenenfalls mit Fluor substituierter C₁-C₆-Alkylrest oder ein C₃-C₆-Cycloalkylrest ist;
R² Wasserstoff oder eine Gruppe ist ausgewählt aus -CH₂C(=CHR)-COOR, -CH₂OCOR, -CH(R)OCOR, -CH(R)OCOOR, -CH(OCOR)OCOR, -CH₂COCH₂OCOR und 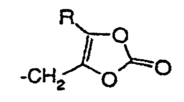 R³ Wasserstoff oder eine Gruppe ausgewählt aus -CH₂C(=CH₂)-COOR, -COOCH₂C(=CHR)-COOR, -COOCH₂OCOR, -COOCH(R)OCOR, -COOCH(R)OCOOR, -COOCH(OCOR)OCOR, -COOCH₂COCH₂OCOR und 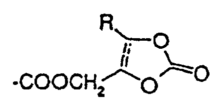 ist, mit dem Vorbehalt, dass einer der Reste R² und R³ Wasserstoff ist und der andere der Reste R² und R³ von Wasserstoff verschieden ist,
R Wasserstoff oder ein C₁-C₆-Alkylrest ist;
R⁴ Wasserstoff oder ein Hydroxyrest ist,
R⁵ Wasserstoff oder ein ω-Hydroxyalkylrest ist und
X CH oder N ist,
ebenso wie pharmazeutisch annehmbare Salze dieser Verbindungen und Hydrate der Verbindungen der Formel I und von deren Salzen.

2. Verbindungen gemäß Anspruch 1 der Formel I-a 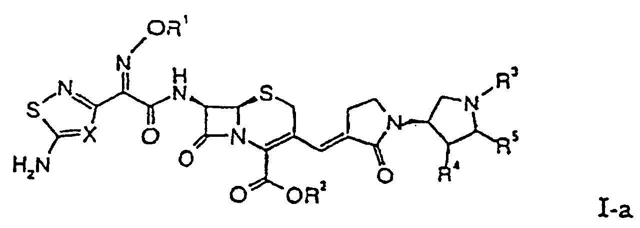 worin R¹, R², R³ und X wie in Anspruch 1 definiert sind und R⁴ und R⁵ Wasserstoff sind,
ebenso wie pharmazeutisch annehmbare Salze dieser Verbindungen und Hydrate der Verbindungen der Formel I-a und von deren Salzen.

3. Verbindungen nach Anspruch 1 oder Anspruch 2, worin R¹ Wasserstoff ist, X N ist, R² Wasserstoff ist und R³ eine Gruppe -COO-CH₂C(=CHCH₂CH₃)-COOCH₂CH(CH₃)₂, -CH₂C(=CH₂) -COOCH₂CH₃ oder 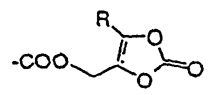 ist.

4. Verbindungen nach Anspruch 1 oder Anspruch 2, worin R¹ und R³ Wasserstoff sind, X N ist und R² -CH₂C(=CHCH₂CH₃) -COOCH₂CH(CH₃)₂, -CH₂C(=CH₂)-COOCH₂CH₃, -CH₂OCOC(CH₃)₃, -CH(CH₃)OCOCH₃, -CH(CH₃)OCOOCH₂CH₃, -CH(OCOCH₃)OCOCH₃, -CH₂COCH₂OCOCH₃ oder 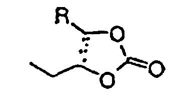 ist.

5. Verbindung nach Anspruch 1 und Anspruch 2, die (6R,7R)-7-[(Z)-2-(5-Amino-[1,2,4]thiadiazol-3-yl)-2-hydroximinoacetylamino]-3-[(E)-(R)-1'-(5-methyl-2-oxo-[1,3]dioxol-4-ylmethoxycarbonyl)-2-oxo-[1,3']bipyrrolidinyl-3-ylidenmethyl]-8-oxo-5-thia-1-azabicyclo[4.2.0]oct-2-en-2-carbonsäure ist.

6. Verbindung nach Anspruch 1 und Anspruch 2, die das Natriumsalz von (6R,7R)-7-[(Z)-2-(5-Amino-[1,2,4]thiadiazol-3-yl)-2-hydroximinoacetylamino]-3-[(E)-(R)-1'-(5-ethyl-2-oxo-[1,3]dioxol-4-ylmethoxycarbonyl)-2-oxo-[1,3']bipyrrolidinyl-3-ylidenmethyl]-8-oxo-5-thia-1-azabicyclo[4.2.0]oct-2-en-2-carbonsäure (1:1) ist.

7. Verbindungen nach einem der Ansprüche 1 bis 6 zur Verwendung als pharmazeutisch aktive Substanzen, insbesondere zur Behandlung und Prophylaxe von infektiösen Krankheiten.

8. Pharmazeutisches Präparat enthaltend eine Verbindung nach einem der Ansprüche 1 bis 6 und einen therapeutisch inerten Träger, insbesondere zur Behandlung und Prophylaxe von infektiösen Krankheiten.

9. Verwendung der Verbindungen nach einem der Ansprüche 1 bis 6 zur Herstellung von Arzneimitteln zur Behandlung und Prophylaxe von infektiösen Krankheiten.

## Revendications

1. Composés de formule I 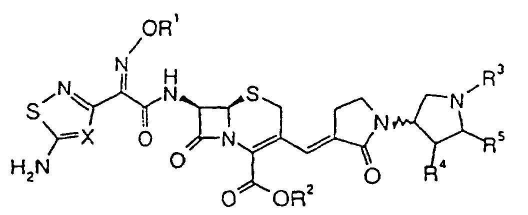 dans laquelle
R¹ est un atome d'hydrogène ou un groupe alkyle en C₁₋₆, éventuellement substitué par un groupe fluoro, ou cycloalkyle en C₃₋₆;
R² est un atome d'hydrogène ou un groupe choisi parmi -CH₂C(=CHR)-COOR, -CH₂OCOR, -CH(R)OCOR, -CH(R)-OCOOR, -CH(OCOR)OCOR, -CH₂COCH₂OCOR et 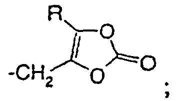
R³ est un atome d'hydrogène ou un groupe choisi parmi -CH₂C(=CH₂)-COOR, -COOCH₂C(=CHR)-COOR, -COOCH₂OCOR, -COOCH(R)OCOR, -COOCH(R)OCOOR, -COOCH(OCOR)OCOR, -COOCH₂COCH₂OCOR, et 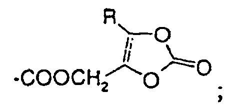 à condition que l'un des radicaux R² et R³ soit un atome d'hydrogène et que l'autre ne soit pas un atome d'hydrogène,
R est un atome d'hydrogène ou un groupe alkyle en C₁₋₆;
R⁴ est un atome d'hydrogène ou un groupe hydroxy,
R⁵ est un atome d'hydrogène ou un groupe ω-hydroxyalkyle; et
X est CH ou N,
ainsi que les sels pharmaceutiquement acceptables desdits composés et les hydrates des composés de formule I et de leurs sels.

2. Composés selon la revendication 1, de formule I-a 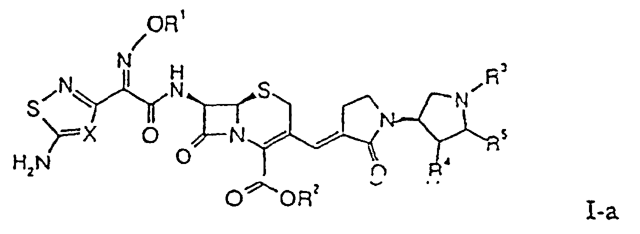 dans laquelle R¹, R², R³ et X sont tels que définis dans la revendication 1 et R⁴ et R⁵ sont des atomes d'hydrogène,
ainsi que les sels pharmaceutiquement acceptables desdits composés et les hydrates des composés de formule I-a et de leurs sels.

3. Composés selon la revendication 1 ou 2, dans lesquels R¹ est un atome d'hydrogène, X est N, R² est un atome d'hydrogène et R³ est un groupe -COO-CH₂C(=CHCH₂CH₃)-COOCH₂CH(CH₃)₂, -CH₂C(=CH₂)-COOCH₂CH₃, ou

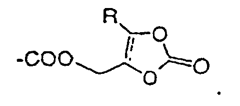

4. Composés selon la revendication 1 ou 2, dans lesquels R¹ et R³ sont des atomes d'hydrogène, X est N et R² est -CH₂C(=CHCH₂CH₃)-COOCH₂CH(CH₃)₂, ou -CH₂C(=CH₂)-COOCH₂CH₃, -CH₂OCOC(CH₃)₃, -CH(CH₃)OCOCH₃, -CH(CH₃)OCOOCH₂CH₃, -CH(OCOCH₃)OCOCH₃, -CH₂COCH₂OCOCH₃, ou

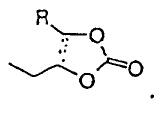

5. Composé selon les revendications 1 et 2, l'acide (6R,7R)-7-[(Z)-2-(5-amino-[1,2,4]thiadiazol-3-yl)-2-hydroxyiminoacétylamino]-3-[(E)-(R)-1'-(5-méthyl-2-oxo-[1,3]dioxol-4-ylméthoxycarbonyl)-2-oxo-[1,3']bipyrrolidinyl-3-ylidèneméthyl]-8-oxo-5-thia-1-azabicyclo[4,2,0]oct-2-ène-2-carboxylique.

6. Composé selon les revendications 1 et 2, le sel de sodium de l'acide (6R,7R)-7-[(Z)-2-(5-amino-[1,2,4]thiadiazol-3-yl)-2-hydroxyiminoacétylamino]-3-[(E)-(R)-1'-(5-éthyl-2-oxo-[1,3]dioxol-4-ylméthoxycarbonyl)-2-oxo-[1,3']bipyrrolidinyl-3-ylidèneméthyl]-8-oxo-5-thia-1-azabicyclo[4,2,0]oct-2-ène-2-carboxylique (1:1).

7. Composés selon l'une quelconque des revendications 1 à 6 à utiliser comme substances actives sur le plan pharmaceutique, en particulier pour le traitement et la prophylaxie de maladies infectieuses.

8. Préparation pharmaceutique contenant un composé selon l'une quelconque des revendications 1 à 6 et un véhicule inerte sur le plan thérapeutique, en particulier pour le traitement et la prophylaxie de maladies infectieuses.

9. Utilisation des composés selon l'une quelconque des revendications 1 à 6 pour la fabrication de médicaments destinés au traitement et à la prophylaxie de maladies infectieuses.
